# EUROPEAN PATENT SPECIFICATION

(11) **EP 1 778 239 B1**
(45) Date of publication and mention of the grant of the patent: **21.08.2013**
(21) Application number: 05762145.0
(22) Date of filing: 18.07.2005
(51) Int. Cl.: A61K 31/52, A61K 31/7076, A61P 27/02

(54) **ADENOSINE A3 RECEPTOR AGONISTS FOR THE TREATMENT OF DRY EYE DISORDERS INCLUDING SJOGREN'S SYNDROME**
ADENOSIN-A3-REZEPTORAGONISTEN ZUR BEHANDLUNG VON TROCKENEM AUGE, EINSCHLIESSLICH SJÖGREN-SYNDROM
AGONISTES DU RECEPTEUR DE L'ADENOSINE A3 POUR LE TRAITEMENT DE TROUBLES DE L'OEIL SEC TELS QUE LE SYNDROME DE SJOGREN

(30) Priority: 28.07.2004 US 591628 P
(43) Date of publication of application: 02.05.2007
(73) Proprietor: Can-Fite Biopharma Ltd., 49170 Petach Tikva (IL)
(72) Inventor: FISHMAN, Pnina, 46365 Herzliya (IL); LORBER, Ilana, 99788 Kefar Shemuel (IL); COHN, Ilan, 46331 Herzliya (IL); REITBLAT, Tatiana, 49170 Petach Tikva (IL)
(74) Representative: Duckworth, Timothy John
(86) International application number: PCT/IL2005/000762
(87) International publication number: WO 2006/011130

(56) References cited:
- WO-A-99/06053
- WO-A-02/083152
- WO-A-2004/029025
- US-A- 5 573 772
- US-A- 5 688 774
- US-A- 5 773 423
- US-A- 5 780 481

## Description

### FIELD OF THE INVENTION

This invention relates to compounds for use in the treatment of Sjogren's syndrome.

### BACKGROUND OF THE INVENTION

The preocular tear film plays an essential role in the maintenance of corneal integrity, the protection against microbial challenge and the preservation of visual acuity. These functions, in turn, are critically dependent upon the stability, tonicity and/or composition of the tear film structure, which includes an underlying mucin foundation, a substantial, middle aqueous component and an overlying lipid layer. Alteration, deficiency or absence of the tear film may lead to intractable desiccation of the corneal epithelium, ulceration and perforation of the cornea, an increased incidence of infectious disease, and ultimately, severe visual impairment and blindness. Throughout the world, countless individuals suffer from tear film dysfunctions, which are collectively diagnosed as keratoconjunctivitis sicca (KCS) or, simply, *dry eye.* By far, the greatest single cause of KCS worldwide, excluding those countries wherein trachoma remains epidemic, is *Sjogren's syndrome.*

Sjogren's syndrome is an autoimmune disorder in which immune cells attack and destroy the glands that produce tears and saliva. Sjogren's syndrome is also associated with lupus, scleroderma, polymyositis and rheumatic disorders such as rheumatoid arthritis. Sjogren's syndrome that results from a rheumatic condition is classified as *secondary Sjogren's syndrome.* The hallmark symptoms of the disorder are dry mouth and dry eyes. In addition, Sjogren's syndrome may cause skin, nose, and vaginal dryness, and may affect other organs of the body including the kidneys, blood vessels, lungs, liver, pancreas, and brain. The ophthalmologic clinical symptoms of Sjogren syndrome are, for example, foreign body sensation, burning, and itching.

There is no known cure for Sjogren's syndrome nor is there a specific treatment to restore gland secretion. Treatment is generally symptomatic and supportive. Moisture replacement therapies may ease the symptoms of dryness. Nonsteroidal anti-inflammatory drugs may be used to treat musculoskeletal symptoms. For individuals with severe complications, corticosteroids or immunosuppressive drugs may be prescribed.

In the scientific literature, reports have suggested that systemic or topical administration of estrogens, cyclosporine A or glucocorticoids might alleviate the ocular manifestations of this disorder. However, other studies indicate that such pharmaceutical exposures are ineffective and, in fact, may accelerate and/or amplify the disease. Indeed, estrogen action may be involved in the etiology of Sjogren's syndrome. Others have suggested that tear stimulants, such as bromhexine or isobutylmethylxanthine, might improve ocular symptoms. These drug effects, though, may be subjective, susceptible to tachyphylaxis and/or limited by the requirement for functional and responsive lacrimal tissue. It has also been proposed that systemic androgen treatment might provide a potential therapy for Sjogren's syndrome and its associated defects. However, a recent report has indicated that systemic androgen therapy is inappropriate for the treatment of the multiple immune dysfunctions in Sjogren's syndrome.

Therefore, the currently prescribed, therapeutic approach for the management of KCS in Sjogren's syndrome is the frequent application of artificial tear substitutes, which permit lubrication of the eye's anterior surface. Unfortunately, this therapy does not represent a cure and does not ameliorate the inherent, ocular immunopathology and resulting KCS associated with this chronic, extremely uncomfortable and vision-threatening disease.

Secondary Sjogren syndrome is a chronic, autoimmune disorder that can be found in a number of other disorders including Rheumatoid Arthritis (RA), Systemic Lupus Erythematosus, scleroderma and other rheumatic disorders. It is characterized mainly by signs of ocular dryness (keratoconjuctivis sicca), dry mouth and other vascular and muscolosceletal manifestations. This disorder is incurable and treatments are focused in the relief of symptoms. Up to 20% of Rheumatoid Arthritis patients have either clinical or subclinical manifestations of secondary Sjogren syndrome.

Adenosine receptors are classified into four major classes: A1, A2a, A2b and A3. A3 adenosine receptors belong to the family of the Gᵢ-protein associated cell surface receptors. Receptor activation leads to its internalization and the subsequent inhibition of adenylyl cyclase activity, cAMP formation and protein kinase A (PKA) expression, resulting in the initiation of various signalling pathways ^{(1,2)}. PKA contains a catalytic subunit PKAc which dissociates from the parent molecule upon activation with cAMP.

WO 2004/029025 discloses methods and compositions for the treatment of autoimmune disorders using clofarabine.

### SUMMARY OF THE INVENTION

The present invention is based on the surprising finding that administration of an A3 adenosine receptor agonist (A3RAg) to a human subject alleviated symptoms of Sjogren's syndrome.

In one embodiment, the invention provides an A3RAg for use in treating or preventing Sjogren's syndrome in an individual wherein said A3RAg is selected from N⁶-2- (4-aminophenyl)ethyladenosine (APNEA), N⁶-(4-amino-3-iodobenzyl) adenosine- 5'-(N-methyluronamide) (AB-MECA), N⁶-(3-iodobenzyl)-adenosine-5'-N-methyluronamide (IB-MECA) and 2-chloro-N⁶-(3-iodobenzyl)- adenosine-5'-N-methyluronamide (Cl-IB-MECA), and physiologically acceptable salts thereof.

In another embodiment the invention provides a pharmaceutical composition for use in the treatment or prevention of Sjogren's syndrome that comprises an effective amount of an A3RAg as specified in the claims and a pharmaceutically acceptable carrier.

In a third embodiment the invention provides use of an A3RAg for the preparation of a pharmaceutical composition for treating or preventing Sjogren's syndrome in an individual, wherein said A3RAg is selected from N⁶-2- (4-aminophenyl)ethyladenosine (APNEA), N⁶-(4-amino-3-iodobenzyl) adenosine- 5'-(N-methyluronamide) (AB-MECA), N⁶-(3-iodobenzyl)-adenosine-5'-N-methyluronamide (IB-MECA) and 2-chloro-N⁶-(3-iodobenzyl)- adenosine-5'-N-methyluronamide (Cl-IB-MECA), and physiologically acceptable salts thereof.

In one preferred embodiment, the A3RAg may be administered topically, for example to the eye or skin. In another preferred embodiment, the A3RAg is administered orally.

The term "***Sjogren's syndrome***" (SS) refers in the context of the present invention to the autoimmune disorder that causes KCS, in which immune cells attack and destroy the glands that produce tears and saliva. In one embodiment of the invention, the term refers to the disorder classified as *secondary Sjogren's syndrome.* In a preferred embodiment, the secondary Sjogren's syndrome results from a rheumatic condition. Symptoms of the disorder may include eye, mouth, skin, nose and vaginal dryness, and may affect other organs of the body including the kidneys, blood vessels, lungs, liver, pancreas, and brain.

In accordance with the invention, means to treat or prevent the ophthalmologic clinical symptom and sign in dry eye including Sjogren syndrome are provided. The ophthalmologic clinical symptom in Sjogren syndrome includes but is not limited to foreign body sensation, burning, and itching; and the ophthalmologic clinical sign in Sjogren syndrome includes but is not limited to corneal and conjunctival erosions stained by fluorescein and rose bengal, and tear film break-up time.

The terms "***treatment***" in the context of the present invention refer to any improvement in the clinical symptoms of the disease, as well as any improvement in the well being of the patients. For example, an improvement may be manifested by a decrease in dry eye symptoms.

The term "***adenosine A3 receptor agonist***" (A3RAg) in the context of the present invention refers to any molecule capable of specifically binding to the adenosine A3 receptor ("A3R"), thereby fully or partially activating said receptor. The A3RAg is thus a molecule that exerts its prime effect through the binding and activation of the A3R. This means that at the doses it is being administered it essentially binds to and activates only the A3R. In a preferred embodiment, an A3RAg has a binding affinity (Kᵢ) to the human adenosine A3 receptor in the range of less than 100 nM, typically less than 50 nM, preferably less than 20 nM, more preferably less than 10 nM and ideally less than 5 nM. Particularly preferred are A3RAgs that have a Kᵢ to the human A3R of less than 2 nM and desirably less than 1 nM.

It should be noted that some A3RAgs can also interact with and activate other receptors with lower affinities (namely a higher Ki). A molecule will be considered an A3RAg in the context of the invention (namely a molecule that exerts its prime effect through the binding and activation A3R) if its affinity to the A3R is at least 3 times (i.e. its Ki to the A3R is at least 3 times lower), preferably 10 times, desirably 20 times and most preferably at least 50 times larger than the affinity to any other of the adenosine receptors (i.e. A1, A2a and A2b).

The affinity of an A3RAg to the human A3R as well as its relative affinity to the other human adenosine receptors (A1, A2a and A2b) can be determined by a number of assays, such as a binding assay. Examples of binding assays include providing membranes containing a receptor and measuring the ability of the A3RAg to displace a bound radioactive agonist; utilizing cells that display the respective human adenosine receptor and measuring, in a functional assay, the ability of the A3RAg to activate or deactivate, as the case may be, downstream signaling events such as the effect on adenylate cyclase measured through increase or decrease of the cAMP level; etc. Clearly, if the administered level of an A3RAg is increased such that its blood level reaches a level approaching that of the Ki of the A1, A2a and A2b adenosine receptors, activation of these receptors may occur following such administration, in addition to activation of the A3R. An A3RAg is thus preferably administered at a dose such that the blood level is such so that essentially only the A3R will be activated.

The characteristic of some adenosine A3 receptor agonists and methods of their preparation are described in detail in, *inter alia,* US 5,688,774; US 5,773,423; US 5,573,772, US 5,443,836, US 6,048,865, WO 95/02604, WO 99/20284, WO 99/06053, WO 97/27173 and applicant's co-pending patent application no. 09/700,751.

When referring to "***physiologically acceptable salts***" of the compounds employed by the present invention it is meant any non-toxic alkali metal, alkaline earth metal, and ammonium salt commonly used in the pharmaceutical industry, including the sodium, potassium, lithium, calcium, magnesium, barium ammonium and protamine zinc salts, which are prepared by methods known in the art. The term also includes non-toxic ***acid addition salts,*** which are generally prepared by reacting the compounds of this invention with a suitable organic or inorganic acid. The acid addition salts are those which retain the biological effectiveness and qualitative properties of the free bases and which are not toxic or otherwise undesirable. Examples include, *inter alia,* acids derived from mineral acids, hydrochloric, hydrobromic, sulfuric, nitric, phosphoric, metaphosphoric and the like. Organic acids include, inter alia, tartaric, acetic, propionic, citric, malic, malonic, lactic, fumaric, benzoic, cinnamic, mandelic, glycolic, gluconic, pyruvic, succinic salicylic and arylsulphonic, e.g. p-toluenesulphonic, acids.

A3RAg which may be employed according to the present invention are N⁶-2-(4-aminophenyl)ethyladenosine (APNEA), N⁶-(4-amino-3-iodobenzyl) adenosine-5'-(N-methyluronamide) (AB-MECA), N⁶-(3-iodobenzyl)-adenosine-5'-N-methyluronamide (IB-MECA) and 2-chloro-N⁶-(3-iodobenzyl)- adenosine-5'-N-methyluronamide (Cl-IB-MECA).

The administration of said A3RAg to a patient may be together with a pharmaceutically acceptable carrier. In the case where the administration is oral, the carrier is one that is acceptable for oral administration. In the case where the administration is topical, the carrier is one that is acceptable for topical administration, one example being ocular administration.

By the term "*pharmaceutically acceptable carrier*" it is meant any one of inert, non-toxic materials, which do not react with the A3RAg and which can be added to formulations as diluents or carriers or to give form or consistency to the formulation. An oral formulation may be in the form of a pill, capsule, in the form of a syrup, an aromatic powder, and other various forms. The carrier is selected at times based on the desired form of the formulation. The carrier may also at times have the effect of the improving the delivery or penetration of the active ingredient to the target tissue, for improving the stability of the drug, for slowing clearance rates, for imparting slow release properties, for reducing undesired side effects etc. The carrier may also be a substance that stabilizes the formulation (e.g. a preservative), for providing the formulation with an edible flavor, etc. The carriers may be any of those conventionally used and is limited only by chemical-physical considerations, such as solubility and lack of reactivity with the A3RAg, and by the route of administration. The carrier may include additives, colorants, diluents, buffering agents, disintegrating agents, moistening agents, preservatives, flavoring agents, and pharmacologically compatible carriers. In addition, the carrier may be an adjuvant, which, by definition are substances affecting the action of the active ingredient in a predictable way. Typical examples of carriers include (a) liquid solutions, where an effective amount of the active substance is dissolved in diluents, such as water, saline, natural juices, alcohols, syrups, etc.; (b) capsules (e.g. the ordinary hard- or soft-shelled gelatin type containing, for example, surfactants, lubricants, and inert fillers), tablets, lozenges (wherein the active substance is in a flavor, such as sucrose and acacia or tragacanth or the active substance is in an inert base, such as gelatin and glycerin), and troches, each containing a predetermined amount of the A3RAg as solids or granules; (c) powders; (d) suspensions in an appropriate liquid; (e) suitable emulsions; (f) liposome formulation; and others.

The term "*effective amount*" in the context of the present invention refers to an amount of A3RAg which results in protection of the patient from the pathological symptoms of Sjogren's syndrome. The "*effective amount*" can be readily determined, in accordance with the invention, by administering to a plurality of tested subjects various amounts of the A3RAg and then plotting the physiological response (for example an integrated "*SS index*" combining several of the therapeutically beneficial effects) as a function of the amount. Alternatively, the effective amount may also be determined, at times, through experiments performed in appropriate animal models and then extrapolating to human beings using one of a plurality of conversion methods; or by measuring the plasma concentration or the area under the curve (AUC) of the plasma concentration over time and calculating the effective dose so as to yield a comparable plasma concentration or AUC. As known, the effective amount may depend on a variety of factors such as mode of administration (for example, oral administration may require a higher dose to achieve a given plasma level or AUC than an intravenous administration); the age, weight, body surface area, gender, health condition and genetic factors of the subject; other administered drugs; etc.

In the following, unless otherwise indicated, dosages are indicated in weight/Kg, meaning weight of administered A3RAg (e.g. IB-MECA or Cl-IB-MECA) per kilogram of body weight of the treated subject in each administration. For example, mg/Kg and microgram/Kg denote, respectively, milligrams of administered agent and micrograms of administered agent per kilogram of body weight of the treated subject.

In mice the effective amount is typically less than about 1000 and preferably less than about 500 microgram/Kg. A typical dose would be in the range of about 1 microgram/Kg to about 200 microgram/Kg, with a preferred dose being in the range of about 5 microgram/Kg to about 150 microgram/Kg. The corresponding effective amount in a human will be a human equivalent amount to that observed in mice, which may be determined in a manner as explained bellow.

The term "*human equivalent*" refers to the dose that produces in human the same effect as featured when a dose of 0.001-1 mg/Kg of an A3RAg is administered to a mouse or a rat. As known, this dose depends and may be determined on the basis of a number of parameters such as body mass, body surface area, absorption rate of the active agent, clearance rate of the agent, rate of metabolism and others.

The human equivalent may be calculated based on a number of conversion criteria as explained bellow; or may be a dose such that either the plasma level will be similar to that in a mouse following administration at a dose as specified above; or a dose that yields a total exposure (namely area under the curve - AUC - of the plasma level of said agent as a function of time) that is similar to that in mice at the specified dose range.

It is well known that an amount of X mg/Kg administered to rats can be converted to an equivalent amount in another species (notably humans) by the use of one of possible conversions equations well known in the art. Examples of conversion equations are as follows:

### Conversion I:

| **Species** | **Body Wt. (Kg)** | **Body Surf. Area (m²)** | **Km Factor** |
|---|---|---|---|
| Mouse | 0.2 | 0.0066 | 3.0 |
| Rat | 0.15 | 0.025 | 5.9 |
| Human Child | 20.0 | 0.80 | 25 |
| Adult | 70.0 | 1.60 | 37 |

Body Surface area dependent Dose conversion: Rat (150g) to Man (70 Kg) is 1/7 the rat dose. This means that, for example, 0.001-1 mg/Kg in rats equals to about 0.14-140 microgram/Kg in humans. Assuming an average human weight of 70 Kg, this would translate into an absolute dosage for humans of about 0.01 to about 10 mg.

### Conversion II:

The following conversion factors: Mouse = 3, Rat = 67. Multiply the conversion factor by the animal weight to go from mg/Kg to mg/m² for human dose equivalent.

| **Species** | **Weight (Kg)** | **BSA (m²)** |
|---|---|---|
| Human | 70.00 | 1.710 |
| Mouse | 0.02 | 0.007 |
| Rat | 0.15 | 0.025 |
| Dog | 8.00 | 0.448 |

According to this equation the amounts equivalent to 0.001-1 mg/Kg in rats for humans are 0.16-64 µg/Kg ; namely an absolute dose for a human weighing about 70 Kg of about 0.011 to about 11 mg, similar to the range indicated in Conversion I.

### Conversion III:

Another alternative for conversion is by setting the dose to yield the same plasma level or AUC as that achieved following administration to an animal. For example, based on measurement made in mice following oral administration of IB-MECA and based on such measurements made in humans in a clinical study in which IB-MECA was given to healthy male volunteers it can be concluded that a dose of 1 microgram/Kg - 1,000 microgram/KG in mice is equivalent to a human dose of about 0.14 - 140 microgram/Kg, namely a total dose for a 70 Kg individual of 0.01 - 10 mg.

Also encompassed within the present invention is a pharmaceutical composition for use in the treatment of Sjogren's syndrome that comprises an effective amount of an A3RAg as defined above and a pharmaceutically acceptable carrier; as well as the use of said A3RAg for the preparation of a pharmaceutical composition for administration to a subject suffering from Sjogren's syndrome and being in need of treatment. In a preferred embodiment, the pharmaceutical composition is formulated for oral use. As will be appreciated, the effective amount in the pharmaceutical composition will depend on the intended therapeutic regimen and the desired therapeutic dose. By way of example, where the dose is 1 mg per day and the desired administration regimen is once daily, the amount of active agent in the pharmaceutical composition will be 1 mg. In cases where it is intended to administer this daily dose in 2 daily administrations, the amount of the active agent in the pharmaceutical composition will be 0.5 mg.

The invention will now be exemplified in the following description of experiments that were carried out in accordance with the invention. It is to be understood that these examples are intended to be in the nature of illustration rather than of limitation. Obviously, many modifications and variations of these examples are possible in light of the above teaching. It is therefore, to be understood that within the scope of the appended claims, the invention may be practiced otherwise, in a myriad of possible ways, than as specifically described hereinbelow.

### Example

The following non-limiting example describes a patient with rheumatoid arthritis (RA) and secondary Sjogren symptoms, mainly manifested by dry eye, treated for years with eye drops (tear substitutes). The patient participated in a phase 2 clinical trial with IB-MECA for the treatment of active RA. The patient's symptoms of dry eye were improved significantly, to the extent of discontinuation of the use of tear substitutes.

***Methods:*** The patient took part in a phase 2, multicenter, randomized, double-blind, parallel-group, dose-ranging study of the safety and preliminary efficacy of daily IB-MECA administered orally for 12 weeks to patients with active rheumatoid arthritis.

The patient is a 53 years old female with a 7 year history of RA, previously treated with 3 disease-modifying anti-rheumatic drugs (DMARDs), and a history of 2-3 years of dry eyes treated with tear substitutes a few times a day. After a 4 week DMARD washout period, the patient was treated with a blinded dose of IB-MECA - either 0.1, 1.0 or 4.0 mg q12h for 12 weeks. She is currently continuing IB-MECA treatment under a long term extension protocol of the original 12 weeks study.

***Results:*** There had been a marked improvement in the condition of the RA in this patient, represented by objective measurements of swollen and tender joints, acute phase reactants (ESR and CRP), patient and physician global assessments, patient assessment of pain and disability. Although the evaluation of secondary Sjogren's symptoms had not been a part of the patient evaluation in this study, the patient reported that after years of continuous use of tear substitutes for dry eye, after about 3-4 weeks of IB-MECA administration she did not need them any more, and she currently has no symptoms of dry eye.

***Conclusion:*** Treatment with IB-MECA resulted in a substantial improvement of the dry eye symptoms of secondary Sjogren's syndrome in a patient with active RA.

## Claims

1. An A3 adenosine receptor agonist (A3RAg) for use in treating or preventing Sjogren's syndrome in an individual wherein said A3RAg is selected from N⁶-2- (4-aminophenyl)ethyladenosine (APNEA), N⁶-(4-amino-3-iodobenzyl) adenosine- 5'-(N-methyluronamide) (AB-MECA), N⁶-(3-iodobenzyl)-adenosine-5'-N- methyluronamide (IB-MECA) and 2-chloro-N⁶-(3-iodobenzyl)- adenosine-5'-N-methyluronamide (Cl-IB-MECA), and physiologically acceptable salts thereof.

2. An A3RAg according to claim 1 for use according to Claim 1, in oral administration.

3. An A3RAg according to claim 1 for use according to Claim 1, in topical application.

4. An A3RAg according to claim 1 for use according to Claim 3, in topical application to the eye.

5. An A3RAg according to claim 1 for use according to any one of Claims 1 to 4, wherein said A3RAg is IB-MECA.

6. An A3RAg as defined in Claim 1 or 5, for use in treating the ophthalmologic clinical symptoms and signs in dry eye disorder including Sjogren's syndrome.

7. An A3RAg according to claim 6 for use according to Claim 6, wherein the ophthalmologic clinical symptom in Sjogren's syndrome is one selected from the group consisting of foreign body sensation, burning, and itching; and the ophthalmologic clinical sign in Sjogren syndrome is one selected from the group consisting of corneal and conjunctival erosions stained by fluorescein and rose bengal, and tear film break-up time.

8. An A3RAg according to claim 6 for use according to Claim 7, wherein said Sjogren's syndrome is secondary Sjogren's syndrome.

9. A pharmaceutical composition for use in the treatment or prevention of Sjogren's syndrome that comprises an effective amount of an A3RAg according to Claim 1 or 5 and a pharmaceutically acceptable carrier.

10. Use of an A3 adenosine receptor agonist (A3RAg) for the preparation of a pharmaceutical composition for treating or preventing Sjogren's syndrome in an individual, wherein said A3RAg is selected from N⁶-2- (4-aminophenyl)ethyladenosine (APNEA), N⁶-(4-amino-3-iodobenzyl) adenosine- 5'-(N-methyluronamide) (AB-MECA), N⁶-(3-iodobenzyl)-adenosine-5'-N-methyluronamide (IB-MECA) and 2-chloro-N⁶-(3-iodobenzyl)- adenosine-5'-N-methyluronamide (Cl-IB-MECA), and physiologically acceptable salts thereof.

11. The use according to Claim 10, wherein the composition is administered orally or topically.

12. The use according to Claim 11, wherein the composition is administered topically to the eye.

13. The use of any one of Claims 10 to 12, wherein said A3RAg is IB-MECA.

14. The use of any one of Claims 10 to 13, for the preparation of a composition for treating the ophthalmologic clinical symptoms and signs in dry eye disorder including Sjogren's syndrome.

15. The use of Claim 14, wherein the ophthalmologic clinical symptom in Sjogren's syndrome is one selected from the group consisting of foreign body sensation, burning, and itching; and the ophthalmologic clinical sign in Sjogren's syndrome is one selected from the group consisting of corneal and conjunctival erosions stained by fluorescein and rose bengal, and tear film break-up time.

## Patentansprüche

1. A3-Adenosinrezeptoragonist (A3RAg) zur Verwendung zur Behandlung oder Vorbeugung von Sjögren-Syndrom bei einem Individuum, wobei der A3RAg ausgewählt ist aus N⁶-2-(4-Aminophenyl)ethyladenosin (APNEA), N⁶-(4-Amino-3-iodbenzyl)adenosin-5'-(N-methyluronamid) (AB-MECA), N⁶-(3-Iodbenzyl)-adenosin-5'-N-methyluronamid (IB-MECA) und 2-Chlor-N⁶-(3-iodbenzyl)-adenosin-5'-N-methyluronamid (Cl-IB-MECA) und physiologisch annehmbaren Salzen davon.

2. A3RAg gemäß Anspruch 1 zur Verwendung gemäß Anspruch 1 zur oralen Verabreichung.

3. A3RAg gemäß Anspruch 1 zur Verwendung gemäß Anspruch 1 zur topischen Anwendung.

4. A3RAg gemäß Anspruch 1 zur Verwendung gemäß Anspruch 1 zur topischen Anwendung auf das Auge.

5. A3RAg gemäß Anspruch 1 zur Verwendung gemäß einem der Ansprüche 1 bis 4, wobei der A3RAg IB-MECA ist.

6. A3RAg wie definiert in Anspruch 1 oder 5 zur Verwendung zur Behandlung der ophthalmologischen klinischen Symptome und Anzeichen bei einer Störung mit trockenem Auge, beinhaltend Sjögren-Syndrom.

7. A3RAg gemäß Anspruch 6 zur Verwendung gemäß Anspruch 6, wobei das ophthalmologische klinische Symptom bei Sjögren-Syndrom eines ist, ausgewählt aus der Gruppe, bestehend aus Fremdkörpergefühl, Brennen und Juckreiz; und das ophthalmologische klinische Anzeichen bei Sjögren-Syndrom eines ist, ausgewählt aus der Gruppe, bestehend aus Hornhaut- und Bindehaut-Erosionen gefärbt durch Fluoreszein und Rose-Bengal und Tränenfilmaufreißzeit.

8. A3RAg gemäß Anspruch 6 zur Verwendung gemäß Anspruch 7, wobei das Sjögren-Syndrom sekundäres Sjögren-Syndrom ist.

9. Pharmazeutische Zusammensetzung zur Verwendung bei der Behandlung oder Vorbeugung von Sjögren-Syndrom, die eine wirksame Menge eines A3RAg gemäß Anspruch 1 oder 5 und einen pharmazeutisch annehmbaren Träger umfasst.

10. Verwendung eines A3-Adenosinrezeptoragonisten (A3RAg) für die Herstellung einer pharmazeutischen Zusammensetzung zur Behandlung oder Vorbeugung von Sjögren-Syndrom bei einem Individuum, wobei der A3RAg ausgewählt ist aus N⁶-2-(4-Aminophenyl)ethyladenosin (APNEA), N⁶-(4-Amino-3-iodbenzyl)adenosin-5'-(N-methyluronamid) (AB-MECA), N⁶-(3-Iodbenzyl)-adenosin-5'-N-methyluronamid (IB-MECA) und 2-Chlor-N⁶-(3-iodbenzyl)-adenosin-5'-N-methyluronamid (Cl-IB-MECA) und physiologisch annehmbaren Salzen davon.

11. Verwendung gemäß Anspruch 10, wobei die Zusammensetzung oral oder topisch verabreicht wird.

12. Verwendung gemäß Anspruch 11, wobei die Zusammensetzung topisch an das Auge verabreicht wird.

13. Verwendung gemäß einem der Ansprüche 10 bis 12, wobei der A3RAg IB-MECA ist.

14. Verwendung gemäß einem der Ansprüche 10 bis 13 zur Herstellung einer Zusammensetzung zur Behandlung der ophthalmologischen klinischen Symptome und Anzeichen bei einer Störung von trockenem Auge beinhaltend Sjögren-Syndrom.

15. Verwendung gemäß Anspruch 14, wobei das ophthalmologische klinische Symptom bei Sjögren-Syndrom eines ist, ausgewählt aus der Gruppe, bestehend aus Fremdkörpergefühl, Brennen und Juckreiz; und das ophthalmologische klinische Anzeichen bei Sjögren-Syndrom eines ist, ausgewählt aus der Gruppe, bestehend aus Hornhaut- und Bindehaut-Erosionen, gefärbt durch Fluoreszein und Rose-Bengal, und Tränenfilmaufreißzeit.

## Revendications

1. Agoniste des récepteurs A3 d'adénosine (AgRA3) pour utilisation dans le traitement ou la prévention du syndrome de Sjögren chez un individu, lequel agoniste AgRA3 est choisi parmi la N⁶-[2-(4-amino-phényl)-éthyl]-adénosine (APNEA), le N⁶-(4-amino-3-iodo-benzyl)-adénosine-5'-(N-méthyl-uronamide) (AB-MECA), le N⁶-(3-iodo-benzyl)-adénosine-5'-(N-méthyl-uronamide) (IB-MECA), et le 2-chloroN⁶-(3-iodo-benzyl)-adénosine-5'-(N-méthyl-uronamide) (Cl-IB-MECA), ainsi que leurs sels physiologiquement admissibles.

2. Agoniste AgRA3 conforme à la revendication 1, pour utilisation selon la revendication 1 en administration par voie orale.

3. Agoniste AgRA3 conforme à la revendication 1, pour utilisation selon la revendication 1 en application locale.

4. Agoniste AgRA3 conforme à la revendication 1, pour utilisation selon la revendication 3 en application locale au niveau de l'oeil.

5. Agoniste AgRA3 conforme à la revendication 1, pour utilisation selon l'une des revendications 1 à 4, lequel agoniste AgRA3 est de l'IB-MECA.

6. Agoniste AgRA3 conforme à la revendication 1 ou 5, pour utilisation dans le traitement de signes et symptômes cliniques ophtalmologiques dans une kérato-conjonctivite sèche, y compris le syndrome de Sjögren.

7. Agoniste AgRA3 conforme à la revendication 6, pour utilisation selon la revendication 6, pour laquelle le symptôme clinique ophtalmologique dans le syndrome de Sjögren est un symptôme choisi dans l'ensemble formé par une sensation de présence d'un corps étranger, une sensation de brûlure et des démangeaisons, et le signe clinique ophtalmologique dans le syndrome de Sjögren est un signe choisi dans l'ensemble formé par des érosions de la cornée et de la conjonctive, mises en évidence par coloration à la fluorescéine et au rose Bengale, et le temps de rupture du film lacrymal.

8. Agoniste AgRA3 conforme à la revendication 6, pour utilisation selon la revendication 7, pour lequel ledit syndrome de Sjögren est un syndrome de Sjögren secondaire.

9. Composition pharmaceutique pour utilisation dans le traitement ou la prévention du syndrome de Sjögren, qui comprend, en un quantité efficiente, un agoniste AgRA3 conforme à la revendication 1 ou 5 et un véhicule pharmacologiquement admissible.

10. Utilisation d'un agoniste des récepteurs A3 d'adénosine (AgRA3) pour la préparation d'une composition pharmaceutique destinée au traitement ou à la prévention du syndrome de Sjögren chez un individu, pour laquelle ledit agoniste AgRA3 est choisi parmi la N⁶-[2-(4-amino-phényl)-éthyl]-adénosine (APNEA), le N⁶-(4-amino-3-iodo-benzyl)-adénosine-5'-(N-méthyl-uronamide) (AB-MECA), le N⁶-(3-iodo-benzyl)-adénosine-5'-(N-méthyl-uronamide) (IB-MECA), et le 2-chloro-N⁶-(3-iodo-benzyl)-adénosine-5'-(N-méthyl-uronamide) (Cl-IB-MECA), ainsi que leurs sels physiologiquement admissibles.

11. Utilisation conforme à la revendication 10, la composition devant être administrée par voie orale ou en application locale.

12. Utilisation conforme à la revendication 11, la composition devant être administrée en application locale au niveau de l'oeil.

13. Utilisation conforme à l'une des revendications 10 à 12, pour laquelle ledit agoniste AgRA3 est de l'IB-MECA.

14. Utilisation conforme à l'une des revendications 10 à 13, pour la préparation d'une composition destinée au traitement de signes et symptômes cliniques ophtalmologiques dans une kérato-conjonctivite sèche, y compris le syndrome de Sjögren.

15. Utilisation conforme à la revendication 14, pour laquelle le symptôme clinique ophtalmologique dans le syndrome de Sjögren est un symptôme choisi dans l'ensemble formé par une sensation de présence d'un corps étranger, une sensation de brûlure et des démangeaisons, et le signe clinique ophtalmologique dans le syndrome de Sjögren est un signe choisi dans l'ensemble formé par des érosions de la cornée et de la conjonctive, mises en évidence par coloration à la fluorescéine et au rose Bengale, et le temps de rupture du film lacrymal.
